# EUROPEAN PATENT SPECIFICATION

(11) **EP 1 726 641 B1**
(45) Date of publication and mention of the grant of the patent: **19.11.2008**
(21) Application number: 06009989.2
(22) Date of filing: 15.05.2006
(51) Int. Cl.: C12N 5/06, B01D 21/01

(54) **Cell separation method and kit using phase separation**
Phasetrennung- verwendendes Zellentrennverfahren und Kit
Méthode de séparation de cellules et kit utilisant la séparation de phase

(30) Priority: 24.05.2005 KR 20050043749
(43) Date of publication of application: 29.11.2006
(73) Proprietor: Samsung Electronics Co., Ltd., Suwon-si, Gyeonggi-Do (KR)
(72) Inventor: Hwang, Kyu-youn 12-131 (18/6), Incheon-si (KR); Lim, Hee-kyun 147-602 Hwanggol Maeul Jugong, Suwon-si Gyeonggi-do (KR); Kim, Joo-ho 103-401 Mujigae Maeul Daelim Apt., Seongnam-si Gyeonggi-do (KR)
(74) Representative: Grünecker, Kinkeldey, Stockmair & Schwanhäusser Anwaltssozietät

(56) References cited:
- WO-A-03/102184
- US-A1- 2005 019 827
- D'SOUZA S F ET AL: "A METHOD FOR THE PREPARATION OF COIMMOBILISATES BY ADHESION USING POLYETHYLENIMINE" ENZYME AND MICROBIAL TECHNOLOGY, vol. 13, no. 6, 1991, pages 508-511, XP002386343 ISSN: 0141-0229
- KAMATH N ET AL: "Immobilization of ureolytic cells through flocculation and adhesion on cotton cloth using polyethylenimine" ENZYME AND MICROBIAL TECHNOLOGY, STONEHAM, MA, US, vol. 13, no. 11, November 1991 (1991-11), pages 935-938, XP009018724 ISSN: 0141-0229

## Description

### BACKGROUND OF THE INVENTION

### 1. Field of the Invention

The present invention relates to a cell separation method and kit using phase separation.

### 2. Description of the Related Art

The separation of cells from mixtures containing unwanted impurities is a challenging problem. This is particularly the case where the cells are present in a culture broth, a biological sample or similar complex mixture, as the methods employed need to allow the capturing of a high proportion of the cells intact, i.e., without killing or lysing the cells, which would cause the release of cellular debris to further contaminate the mixture. This means that the reagents used in cell concentration and separation steps must capture the cells very efficiently over a range of cell densities, and not interfere by lysing the cell walls or making the cells to "leaky" to nucleic acid before they are separated from the mixture. Also, the reagents used should not interfere with downstream processes using the cells, the recovering of nucleic acid from the cells and/or the processing of the nucleic acid.

There are a number of known methods of binding cells to a solid support. For example, non-specific binding of the cells to a support may be achieved by appropriately choosing the solid support and conditions e.g., the chemical or physical nature of the surface of the solid support (e.g., hydrophobicity or charge), the pH or composition of the isolation medium, etc. The nature of the target cells may also play a role and it has been shown, for example, that certain hydrophobic cells may be readily bound non-specifically to hydrophobic surfaces, whereas hydrophilic cells may be readily bound to hydrophilic surfaces. Negatively charged cells such as B-lymphocytes have also been observed to have a high degree of non-specific binding to slightly-positively charged surfaces. Thus solid supports having appropriately charged surfaces for binding of a desired cell type may be used. Appropriate buffers may be used as media for cell separation to achieve conditions appropriate for cell binding by simply placing the solid support and the sample in contact in the buffer. Conveniently, a buffer having an appropriate charge and osmotic pressure may be added to a sample containing cells before, after, or during the placing of the sample in contact with a solid support.

U.S. Patent No. 6,617,105 discloses a method of separating nucleic acid from a sample containing cells which includes: binding cells in a sample to a solid support coated with cell-binding moieties; and lysing the cells bound to the solid support. In this patented method, isopropanol and 0.75 M ammonium acetate are used as cell flocculating agents. However, this patented method does not disclose use of a kosmotropic salt having a predetermined pH to efficiently separate cells, which characterizes the present invention.

International Publication No. WO 03/102184 A1 discloses a method of separating cells containing target nucleic acid comprising: placing a mixture containing cells in contact with a flocculating agent capable of aggregating the cells and with a solid phase capable of binding the cells, wherein the flocculating agent is polyamine or a cationic detergent; separating the aggregated cells from the mixture using the solid phase; and purifying the target nucleic acid from the cells. This patented method is characterized by separating cells containing target nucleic acid using a cell flocculating agent such as polyamine, but does not disclose use of a kosmotropic salt - triggered phase separation having a predetermined pH for efficiently separating cells, which characterizes the present invention.

If the concentration of cells or viruses is very low at an early stage of purification of nucleic acid from the cells or the viruses, the cells or the viruses must be enriched. Since the volume of samples used in miniaturized chips is generally very low, it is necessary to carry out research on cell enrichment.

The inventors of the present invention has discovered, while conducting research on ways to separate cells or viruses from a mixture, that the efficiency of cell or virus separation can be increased by using a kosmotropic salt - triggered phase separation of cationic polymer, a certain pH, and a solid substrate and has completed the present invention.

### SUMMARY OF THE INVENTION

The present invention provides a cell or virus separation method using phase separation.

The present invention also provides a cell or virus separation kit using phase separation.

According to an aspect of the present invention, there is provided a method of separating cells or viruses from a mixture using phase separation. The method includes: suspending a sample containing cells or viruses in a kosmotropic salt solution having a pH of 3-6, wherein the kosmotropic salt is citrate or phosphate; separating the cells or viruses by adding a cationic polymer to the suspension; adhering the cells or viruses to a solid substrate by phase separation of cationic polymer; and separating the solid substrate on which the cells or viruses are adhered from the suspension.

According to another aspect of the present invention, there is provided a kit for separating cells or viruses from a mixture. The kit includes: a cationic polymer;
a kosmotropic salt solution having a pH of 3-6; and a solid substrate to which the cell-cationic polymer complex adheres.

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic diagram illustrating a method of separating cells or viruses from a mixture using phase separation according to an exemplary embodiment of the present invention;
FIG. 2 is a diagram illustrating various phase separation patterns according to the type of kosmotropic salt;
FIG. 3 presents optical microscope photos for illustrating various cell adherence patterns of E. coli cells according to the type of kosmotropic salt, according to an exemplary embodiment of the present invention;
FIG. 4 illustrates various phase separation patterns according to pH;
FIG. 5 illustrates various cell adherence patterns of E. coli cells according to pH;
FIG. 6 illustrates various phase separation patterns according to the concentration of a PEI solution;
FIG. 7 illustrates various cell adherence patterns of E. coli cells according to the concentration of a PEI solution;
FIG. 8 illustrates various cell adherence patterns of E. coli cells according to the type of a solid substrate;
FIG. 9 presents various optical microscope photos for illustrating various cell adherence patterns obtained using a flow control system; and
FIG. 10 presents optical microscope photos for illustrating different cell adherence patterns of E. coli cells according to whether the E. coli cells are treated with a NaOH solution.

### DETAILED DESCRIPTION OF THE INVENTION

The present invention relates to a method of separating cells using a kosmotropic salt solution having a predetermined pH, a cationic polymer, and a solid substrate, wherein the kosmotropic salt is citrate or phosphate.

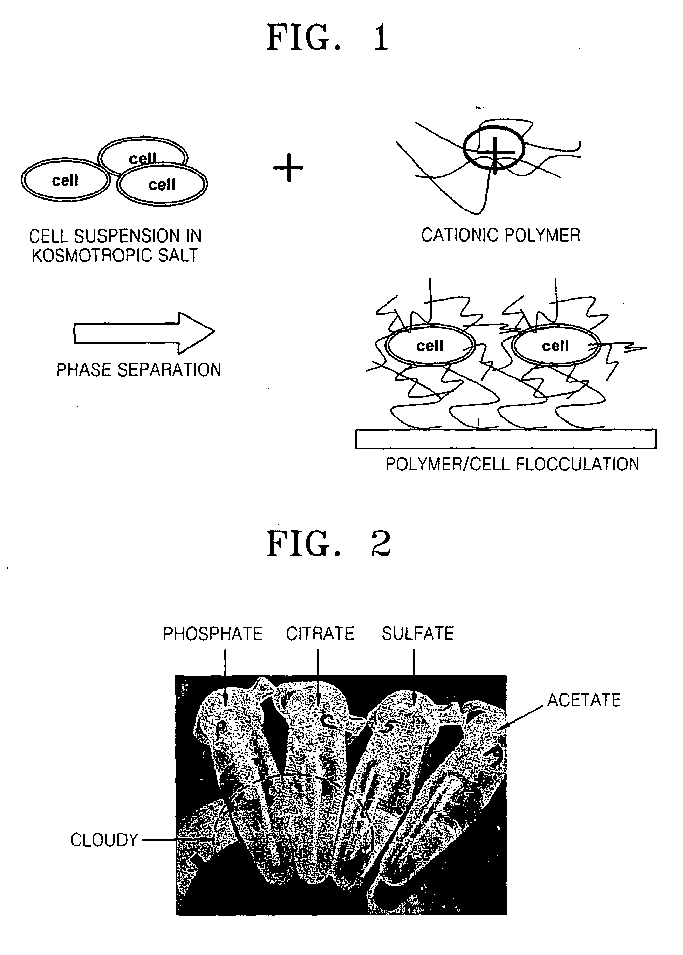

FIG. 1 is a schematic diagram illustrating a cell or virus separation method according to an exemplary embodiment of the present invention.

In detail, cells or viruses are suspended in a kosmotropic salt solution having a predetermined pH, and a cell separation agent such as polyamines is added to the kosmotropic salt solution. Then, the cells or viruses present in the suspension aggregate and separate with the aid of phase separation of polyamine. Thereafter, a solid substrate is placed in contact with the suspension. Then, the aggregated cells or viruses - polymer complex adhere to the solid substrate. When the solid substrate is separated from the suspension, the concentrated cells or viruses are separated from the suspension.

The method of separating cells or viruses from a mixture includes suspending a sample containing cells or viruses in a kosmotropic salt solution having a pH of 3-6. The cells or viruses must be suspended in a kosmotropic salt solution and a cell separating agent must be added to the suspension to induce the phase separation and adherence of a high concentration of cells or viruses to a solid substrate. In the present invention, the pH range of the kosmotropic salt solution is very important. As is apparent from an embodiment of the present invention which will be described below, the efficiency of adhering cells or viruses to a solid substrate considerably decreases when the kosmotropic salt solution has a pH outside the range of 3-6.

Also, the type of kosmotropic salt used to suspend cells or viruses is very important. Once dissolved in water, a kosmotropic salt reduces the solubility of other substances present in the solution. The efficiency of aggregating cells or viruses in a kosmotropic salt solution may vary according to the type of kosmotropic salt present in the kosmotropic salt solution. For example, cells or viruses suspended in a kosmotropic salt solution such as a citrate or phosphate solution flocculate properly. On the other hand, cells or viruses suspended in a kosmotropic salt solution such as an acetate or sulfate solution do not flocculate properly.

The method of separating cells or viruses from a mixture also includes aggregating the cells or viruses in the suspension by adding a cationic polymer to the suspension. In detail, when a cationic polymer is added to the suspension, the cells or viruses present in the suspension may aggregate properly and then phase separation occurs.

The method of separating cells or viruses from a mixture also includes adhering the aggregated cells or viruses - polymer complex to a solid substrate by placing the solid substrate in contact with the suspension. In detail, when a solid substrate is placed in contact with the suspension, the aggregated cells or viruses adhere to the solid substrate.

The method of separating cells or viruses from a mixture also includes separating the solid substrate from the suspension. In detail, the cells or viruses suspended in the kosmotropic salt solution aggregate and adhere to the solid substrate while phase separation occurs, therefore, when the solid substrate is separated from the suspension, and the concentrated cells or viruses are separated from the suspension.

In an embodiment of the present invention, the cationic polymer may be polyamine, which is a substance having one or more covalently linked units, each unit having one or more amine groups, e.g., primary, secondary, tertiary, quaternary, aromatic or heterocyclic amine groups, which are positively charged at the pH at which the cationic polymer is used in a cell separation method. In an embodiment of the present invention, the polyamine may comprise a plurality of covalently linked units. The units forming the polyamine may be the same or different. In addition to the amine groups, the polyamine may be either unsubstituted or substituted with one or more further functional groups. Examples of the polyamine include polyamino acid, polyallylamine, polyalkylimine such as polyethylenimine, a polymerized biological buffer containing amine groups, and polyglucoseamine. All of these examples may be either substituted or unsubstituted.

If the polyamine is polyamino acid, linked amino acids forming the polyamino acid may be the same or different. Examples of the polyamino acid include poly-lysine and poly-histidine. The amino acids forming the polyamino acid may be D amino acids, L amino acids, or a mixture of D and L amino acids.

If the polyamine is polyallylamine or polyallylamine.HCl, the polyallylamine may be represented by the following formula:

Poly(allylamine Hydrochloride): [-CH₂CH(CH₂NH₂-HCl)-]ₙ,

or

Poly(allylamine): [-CH₂CH(CH₂NH₂)-]ₙ

where n is at least 3. The polyallylamine may be unsubstituted or may have one or more substitutions in addition to those presented in the above formula. The polyallylamine can be produced by polymerizing 2-propen-1-amine or a similar monomer comprising alkene and amine functional groups. Examples of the polyallylamine include polyallylamines supplied by Aldrich in the form of either solids or solutions (e.g., 20 wt% solutions). Examples of the polyallylamine include poly(allylamine) reference 47,914-4 (20 wt% solution, Mw ca 65,000), poly(allylamine) reference 47,913-6 (20 wt% solution, Mw ca 17,000), poly(allylamine hydrochloride) reference 28,321-5 (solid, Mw ca 15,000), and poly(allylamine hydrochloride) reference 28,322-3 (solid, Mw ca 70,000), which are all disclosed in the 2001 Aldrich Catalogue, page 1385.

If the polyamine is a polyalkylimine such as polyethylenimine (PEl), it may be represented by the following formula:
Polyethylenimine: (-NHCH₂CH₂-)ₓ[-N(CH₂CH₂NH₂)CH₂CH₂-]_{y}.

The polyallylamine may be a polymerized biological buffer such as poly Bis-Tris. Examples of biological buffers which have amine groups and can be polymerized include Bis-2-hydroxyethyliminotrishydroxymethylmethane (Bis-Tris), pKa 6.5; 1,3-bistrishydroxymethylmethylaminopropane (Bis-Tris propane), pKa 6.8; N-trishydroxymethylmethylglycine (TRICINE), pKa 8.1; and Trishydroxymethylaminomethane (TRIS), pKa 8.1.

The polyamine may be a polyglucosamine such as chitosan, which is a readily available material derived from the shells of crustacea and formed from repeating units of D-glucosamine.

In an embodiment of the present invention, the placing of the solid substrate in contact with the suspension may be performed in a static state or a fluidic state. In detail, the solid substrate may be placed in contact with the suspension in a static state or a fluidic state. In other words, the solid substrate may be placed in contact with the suspension while causing the suspension to flow with the aid of a flow control system. The solid substrate may be planar. Alternatively, the solid substrate may have a pillar structure so that it has a sufficiently large surface area to efficiently capture the cells or viruses present in the suspension when used together with the flow control system.

In an embodiment of the present invention, the solid substrate may have a planar structure, a bead structure, or a pillar structure. The solid substrate may be formed of any material to which cells or viruses can adhere. However, the solid substrate must not be formed of a material that dissolves in the suspension.

Conveniently, the solid substrate may be formed of glass, silica, latex, or a polymeric material. The solid substrate may be formed of a material that can offer a sufficiently large surface area to capture as many cells as possible. The solid substrate may be formed of, for example, a porous material or fine particles, and thus have uneven surfaces.

In an embodiment of the present invention, examples of the cells or viruses present in the suspension include bacteria, bacteriophage, plant cells, animal cells, plant viruses and animal viruses.

In an embodiment of the present invention, the kosmotropic salt may be citrate or phosphate. Not all kosmotropic salts can bring about phase separation, only citrate and phosphate buffers. The objectives of the present invention can be fully achieved only when using certain kinds of kosmotropic salts.

In an embodiment of the present invention, the cationic polymer may be coupled to, mixed with, or associated with the solid substrate. The cationic polymer facilitates the separation of the cells or viruses present in the suspension by its phase separation, thereby making it easy to separate the cells or viruses from the suspension.

In an embodiment of the present invention, the cationic polymer may be initially soluble and thus aggregate with the cells or viruses present in the suspension and then separated from the suspension by kosmotropic salts , thereby enabling the cells or viruses to separate from the suspension. For example, the cationic polymer may be dissolved in water, and the resulting solution may be added to the suspension. Then, the cationic polymer aggregates together with the cells or viruses present in the suspension, and the cell - polymer complex adheres to the solid substrate by its phase separation when the solid substrate is placed in contact with the suspension.

The present invention also provides a kit for separating cells or viruses which comprises: a cationic polymer; a kosmotropic salt solution having a pH of 3-6; and a solid substrate to which the cell-cationic polymer complex can adhere.

In detail, the kit for separating cells or viruses comprises a cationic polymer which can facilitate the aggregation of cells or viruses, a kosmotropic salt solution having a pH of 3-6 in which cells or viruses are suspended; and a solid substrate to which the cell-cationic polymer can adhere.

In an embodiment of the present invention, the solid substrate may be planar. Alternatively, the solid substrate may comprise a plurality of pillars so that it has a sufficiently large surface area to capture as many cells or viruses as possible.

In an embodiment of the present invention, examples of the cells or viruses present in the suspension include bacteria, bacteriophage, plant cells, animal cells, plant viruses, and animal viruses.

In an embodiment of the present invention, the kosmotropic salt present in the kosmotropic salt solution may be a citrate or a phosphate. Not all kosmotropic saltscan bring about phase separation, only citrate and phosphate buffers. The objectives of the present invention can be fully achieved only when using certain kinds of kosmotropic salts.

In an embodiment of the present invention, the cationic polymer may be polyamine which is a substance having one or more covalently linked units, each unit having one or more amine groups, e.g., primary, secondary, tertiary, quaternary, aromatic or heterocyclic amine groups, which are positively charged at the pH at which the cationic polymer is used in a cell separation method. In an embodiment of the present invention, the polyamine may comprise a plurality of covalently linked units. The units forming the polyamine may be the same or different. In addition to the amine groups, the polyamine may be either unsubstituted or substituted with one or more further functional groups. Examples of the polyamine include polyamino acid, polyallylamine, polyalkylimine such as polyethylenimine, a polymerized biological buffer containing amine groups, and polyglucosamine. All of these examples may be either substituted or unsubstituted.

The present invention will now be described in greater detail with reference to the following examples. The following examples are for illustrative purposes only and are not intended to limit the scope of the invention.

### Example 1: Phase Separation Using Kosmotropic Salt

4 types of kosmotropic salts were tested to determine whether they would bring about phase separation. 0.1 M sodium phosphate (pH 4), 0.1 M sodium citrate (pH 4), 0.1 M sodium acetate (pH 4), and 0.1 M sodium sulfate (pH 4) were used as the 4 kosmotropic salts. Branched polyethyleneimine (PEl) (Mw ca 750,000) dissolved in deionized water to a concentration of 2.5 mg/ml was used as a cationic polymer. 1 50 µl of each of the 4 kosmotropic salts was mixed with 150 µl of the PEI solution in an Eppendorf tube. FIG. 2 is a photograph illustrating phase separation in each of these mixtures. Referring to FIG. 2, the mixture of sulfate and PEI and the mixture of acetate and PEI are clear, while the mixture of citrate and PEI and the mixture of phosphate and PEI are cloudy. Accordingly, not all kosmotropic salts can bring about phase separation, only citrate and phosphate.

### Example 2: Phase Separation of E. coli cells using Kosmotropic Salt

An experiment for determining whether phase separation occurs in a kosmotrpic salt solution containing E. coli cells was carried out. In the experiment, a 0.1 M sodium phosphate solution (pH 4), a 0.1 M sodium citrate solution (pH 4), a 0.1 M sodium acetate solution (pH 4), and a 0.1 M sodium sulfate solution (pH 4) were used as kosmotropic salts. E. coli cells BL21 (2 ×10⁷ cells/ml) were used. The E. coli cells were suspended in each of the kosmotropic salt solutions. Branched polyethyleneimine (PEI) (Mw ca 750,000) dissolved in deionized water to a concentration of 2.5 mg/ml was used as a cationic polymer. 150 µl of each of the 4 kosmotropic salt solutions was mixed with 150 µℓ of the PEI solution. A carboxyl-coated substrate was used as a solid substrate. In detail, a 60 µℓ patch was attached to the carboxyl-coated substrate, and then 60 µℓ of a mixture of the cell suspension and the PEI solution was applied to the carboxyl-coated substrate. The carboxyl-coated substrate was incubated for 5 minutes at room temperature and then rinsed once with 35 µℓ of the suspension (pH 4) for 1 minute. The carboxyl-coated substrate was stained with gram staining solution for E. coli cells, which is known to one of ordinary skill in the art, in order to stain E. coli cells attached to the carboxyl-coated substrate. In detail, a crystal violet solution was applied to the carboxyl-coated substrate such that portions of the carboxyl-coated substrate on which E. coli cells were attached could be soaked with the crystal violet solution, and 1 minute later, the carboxyl-coated substrate was rinsed with running water. Thereafter, the carboxyl-coated substrate was treated with gram iodine solution, gram bleacher, or gram safranin solution such that the E. coli cells attached to the carboxyl-coated substrate could be gram-stained. Thereafter, the carboxyl-coated substrate was dried at room temperature and then photographed at ×450 or x3000 magnification using an optical microscope.

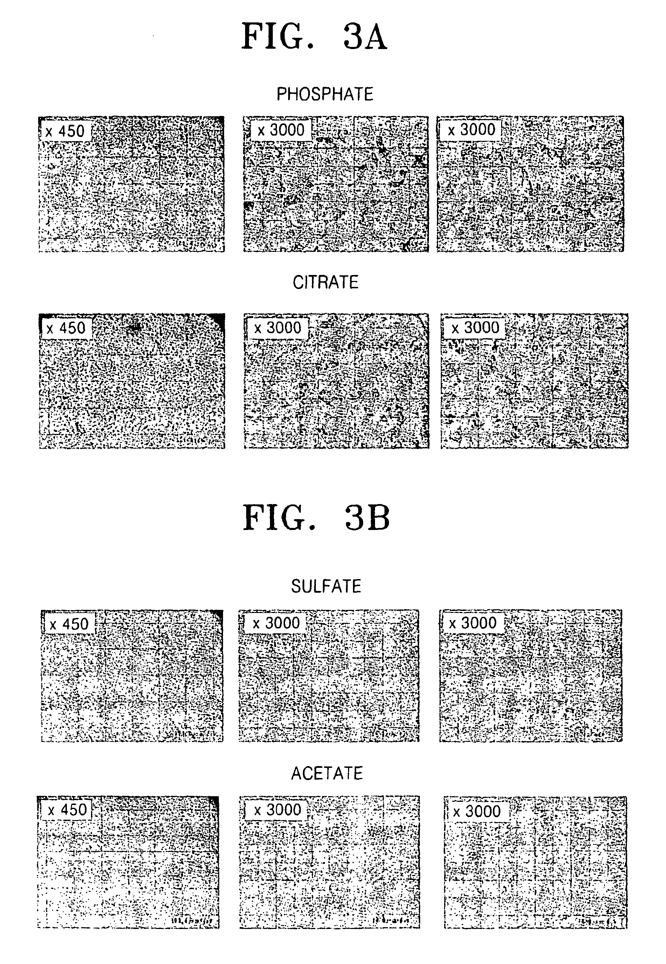

FIG. 3 presents optical microscope photos illustrating various cell adherence patterns of E. coli cells for various types of kosmotropic salts according to an exemplary embodiment of the present invention. Referring to FIG. 3A, a high concentration of E. coli cells was evenly attached to the entire surface of a solid substrate when a citrate solution and a phosphate solution were used and when a phosphate solution was used. Thus, citrate and phosphate were found to cause phase separation. Ten times the E. coli cells attaches to a solid substrate in an embodiment of the present invention using citrate or phosphate than in a method involving cell binding using a hydrophobic interaction in a static system. On the other hand, referring to FIG. 3B, sulfate and acetate resulted in little cell adherence. Thus, phase separation results in an increase in the number of cells adhered to a solid substrate.

### Example 3: Influence of pH on Phase Separation

In order to determine the influence of pH on phase separation, sodium phosphate (pH 3-8) was used. In the present example, experiments were carried out under the same conditions as in Example 1 except that sodium phosphate (pH 3, 4, 5, 7, and 8) was used as kosmotropic salts.

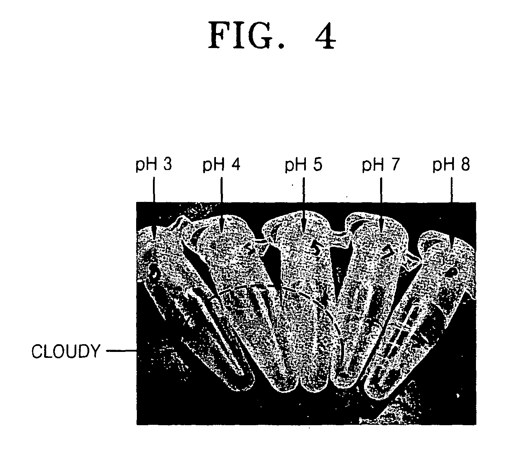

FIG. 4 is a photograph illustrating various phase separation patterns according to pH. Referring to FIG. 4, phase separation occurred only at pH 3-5. Therefore, the occurrence of phase separation is affected by both the type of kosmotropic salt and the certain change of cationic polymer according to pH.

### Example 4: Influence of pH on Cell Adherence

In order to determine the influence of pH on cell adherence, sodium phosphate (pH 3-7) was used. In the present example, experiments were carried out under the same conditions as in Example 2 except that sodium phosphate (pH 3, 4, 5, and 7) was used as kosmotropic salts.

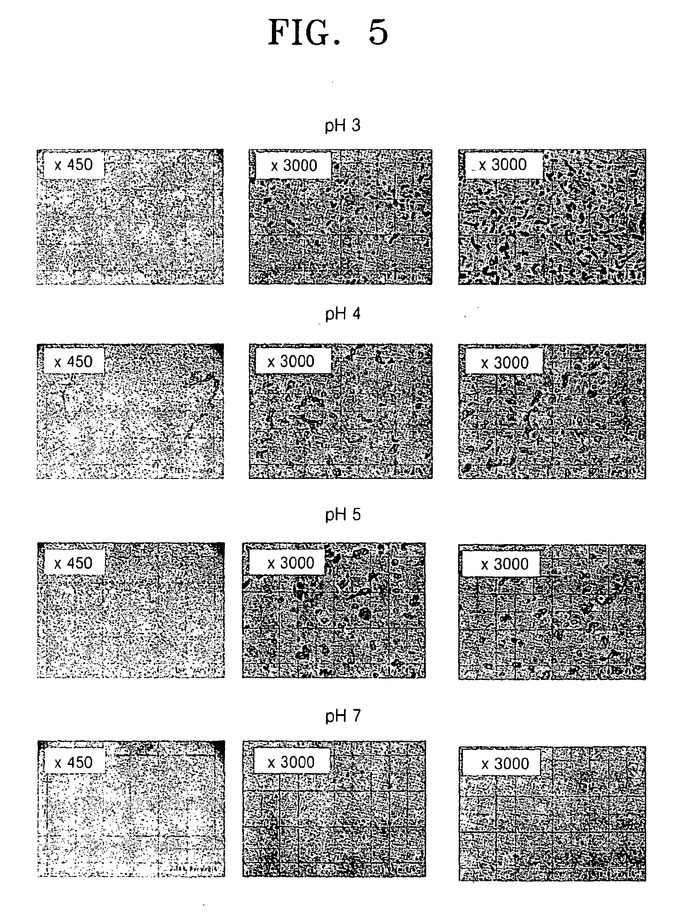

FIG. 5 presents optical microscope photos illustrating various cell adherence patterns of E. coli cells according to pH. Referring to FIG. 5, cell adherence only occurred at pH 3-5, the pH range at which phase separation occurs. Therefore, phase separation induced by a cationic polymer directly affects cell adherence.

### Example 5: Influence of PEI Concentration on Phase Separation

In order to determine the influence of the concentration of a PEI solution on phase separation, a PEI solution having a concentration of 0.1-25 mg/ml in deionized water was used. In the present example, experiments were carried out under the same conditions as in Example 3 except that 0.1 M sodium phosphate (pH 4) was used as a kosmotropic salt.

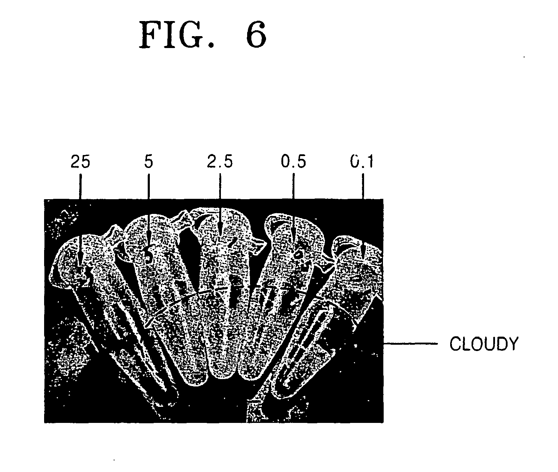

FIG. 6 is a photograph illustrating various phase separation patterns according to the concentration of a PEI solution. Referring to FIG. 6, phase separation only occurred when using PEI solutions each having concentrations of 0.1, 0.5, 3, and 5 mg/ml in deionized water. In addition, phase separation was more apparent when using the PEI solutions each having concentrations of 3 and 5 mg/ml in deionized water than when using the PEI solutions having concentrations of 0.1 and 0.5 mg/ml in deionized water. If the concentration of sodium phosphate is greater than 0.1 M, phase separation is expected to occur even when using a PEI solution having a concentration of 25 mg/ml in deionized water.

### Example 6: Influence of PEI Concentration on Cell Adherence

In order to determine the influence of the concentration of a PEI solution on cell adherence, a PEI solution having a concentration of 0.1 - 25 mg/ml in deionized water was used. In the present example, experiments were carried out under the same conditions as in Example 4, except that 0.1 M sodium phosphate (pH 4) was used as a kosmotropic salt.

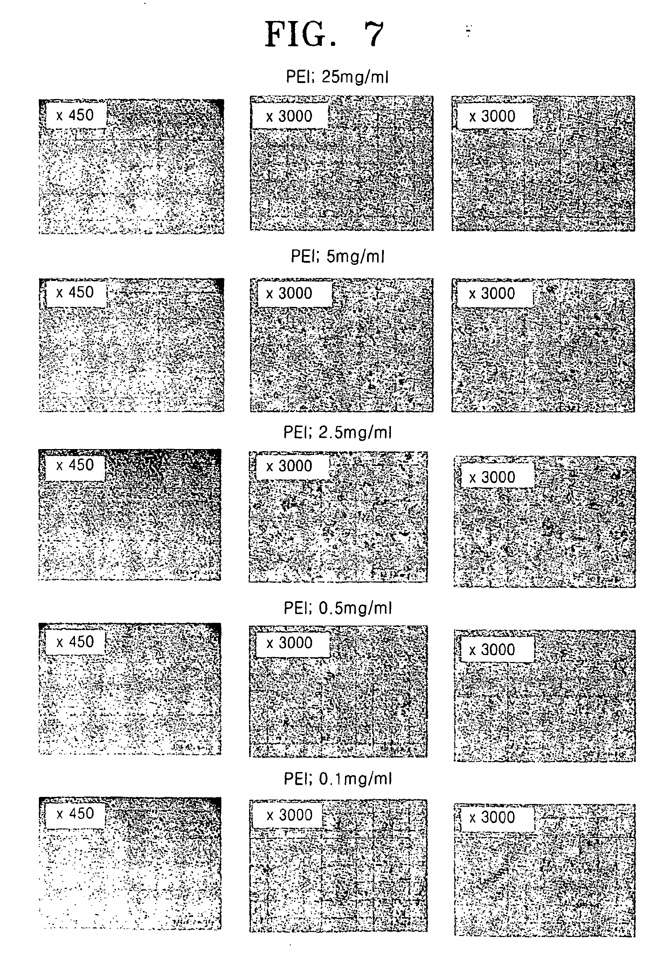

FIG. 7 presents optical microscope photos illustrating various cell adherence patterns according to the concentration of a PEI solution. Referring to FIG. 7, cell adherence only occurred when using a PEI solution having a concentration of 0.5-5 mg/ml in deionized water. Since the occurrence of cell adherence is closely related to the concentration of kosmotropic salt, cell adherence is expected to occur even when using a PEI solution having a concentration of 25 mg/ml in deionized water if the concentration of kosmotropic salt is greater than 0.1 M.

### Example 7: Influence of Type of Solid Substrate on Cell Adherence

In order to determine the influence of the type of solid substrate on cell adherence, an amine-coated solid substrate which was positive charge-coated using gamma aminopropyltriethoxysilane (GAPS) and a carboxyl-coated solid substrate which was negative charge-coated were used. In the present example, experiments were carried out under the same conditions as in Example 6 except that PEI solutions each having concentrations of 0, 0.5, and 2.5 mg/ml in deionized water were used.

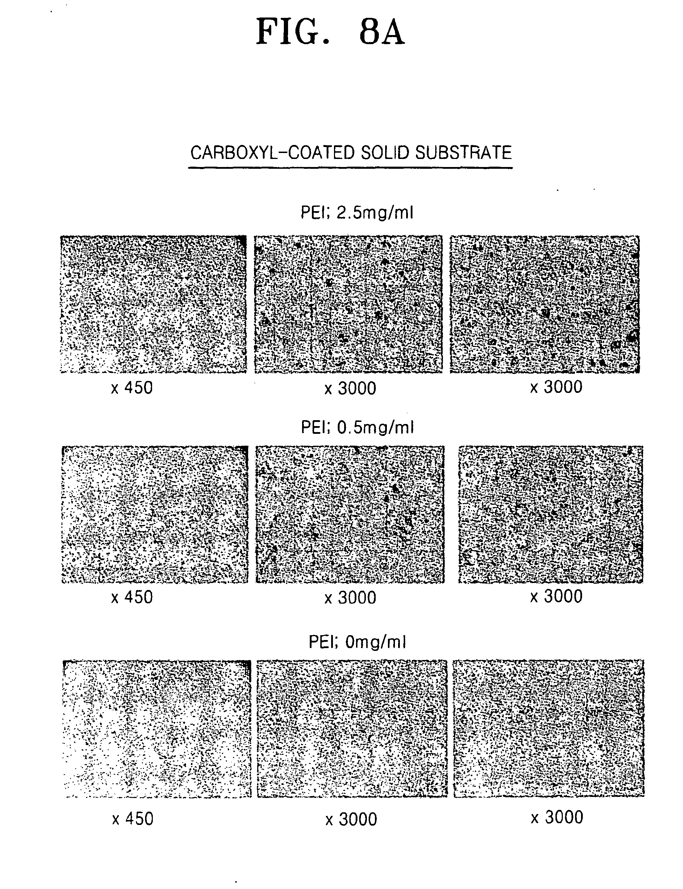

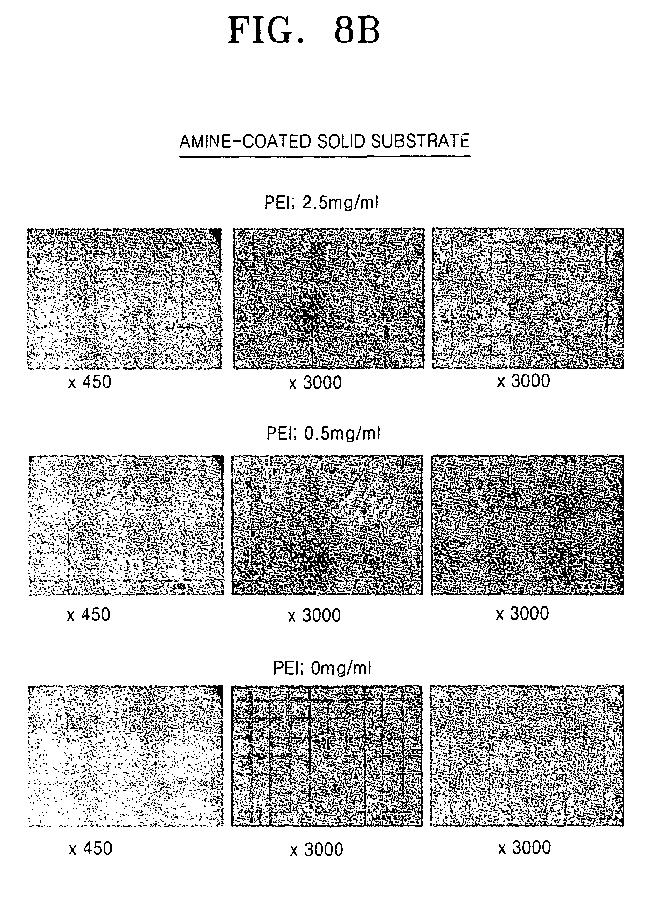

FIG. 8 presents optical microscope photographs illustrating various cell adherence patterns for various types of solid substrates. Referring to FIG. 8A, a high concentration of E. coli cells were adhered to the negatively-charged carboxyl-coated solid substrate at all the PEI concentrations but a PEI concentration of 0 mg/ml. Referring to FIG. 8B, the cell adherence efficiency of the positively-charged amine-coated solid substrate was lower than the cell adherence efficiency of the negatively-charged carboxyl-coated solid substrate at all the PEI concentrations.

### Example 8: Separation of E. coli cells Using Flow control system

Experiments were carried out to determine whether cell adherence occurs in a fluidic control system. In the experiments, an amine-coated solid substrate which was positive charge-coated using GAPS, a carboxyl-coated solid substrate which was negative charge-coated, and a hydrophobic-coated solid substrate which was coated with octadecyldimethyl (3- trimethoxysilylpropyl) ammonium chloride (OTC) were used. The experiments were carried out under the same conditions as in Example 7 except that a PEI solution having a concentration of 2.5 mg/ml in deionized water was used.

The experiments were carried out by flowing 200 µℓ of E. coli cells through the flow control system only one time at a speed of 0.3 cm/sec (400 µℓ/min) using a syringe pump (Harvard, PHD2000). The flow control system had a total surface area of 5 mm×17.3 mm, a cross sectional area of 2.5 mm² (= 5 mm×0.5 mm), and an aspect ratio of 10:1 or higher. Then, the flow control system was rinsed by flowing a sodium phosphate buffer solution (pH 4) through the flow control system at a flow rate of 400 µℓ/min.

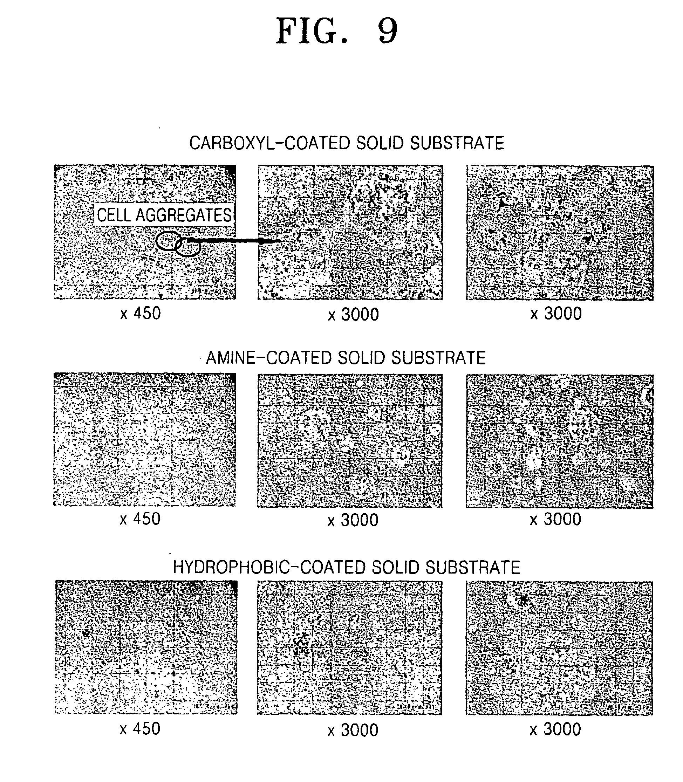

FIG. 9 presents optical microscope photos illustrating various cell adherence patterns of E. coli cells for various types of solid substrates in a flow control system. Referring to FIG. 9, E. coli cells adhered to the solid substrate are rarely discernible at ×450 magnification but can be clearly observed at x3000 magnification in a rod shape. Therefore, E. coli cells can be easily and efficiently adhered to a solid substrate when using a flow control system instead of a static system, and that the cell adherence efficiency of a carboxyl-coated solid substrate is higher than those of an amine-coated solid substrate and a hydrophobic-coated solid substrate.

### Example 9: On-Chip Lysis Experiments

Experiments were carried out to determine whether E. coli cells separated on-chip are efficiently lyzed. In the experiments, an amine-coated solid substrate which was positive charge-coated using GAPS was used. The experiments were carried out under the same conditions as in Example 7 except that a PEI solution having a concentration of 2.5 mg/ml in deionized water was used.

In detail, the experiments were carried out by adhering E. coli cells to a chip through the above-mentioned method using a 60 µℓ patch and treating one of 2 chambers of a 30 µℓ patch with a 0.1 N NaOH solution for 2 minutes while leaving the other chamber of the 30 µℓ patch untreated.

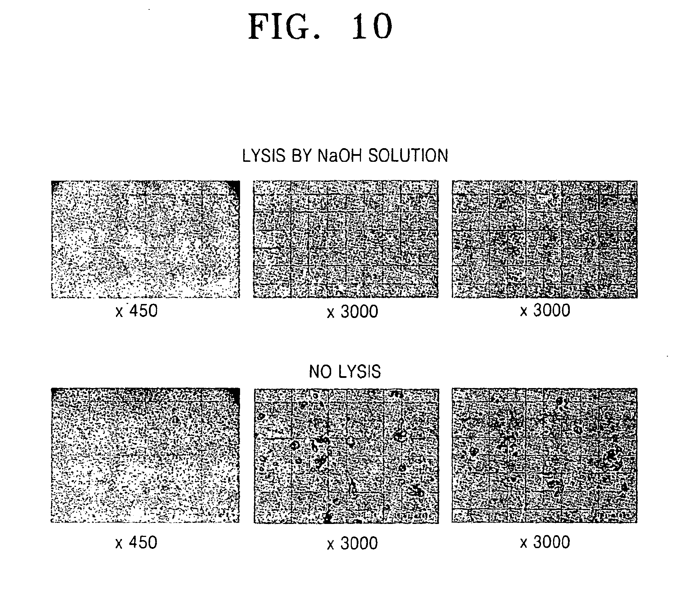

FIG. 10 presents optical microscope photos illustrating various cell adherence patterns of E. coli cells according to whether the E. coli cells are treated with a NaOH solution. Referring to FIG. 10, when treated with a 0.1 N NaOH solution, E. coli cells were easily lyzed such that little E. coli cells could be detected from a solid substrate. On the other hand, when not treated with the 0.1 N NaOH solution, E. coli cells were rarely lyzed such that they were detected from a solid substrate as they were. Therefore, cells separated using the cell separation method and apparatus according to the present invention can be easily lyzed. Thus, the cell separation method and apparatus according to the present invention can be useful for post-cell separation processes such as nucleic acid purification.

As described above, according to the present invention, it is possible to separate a high concentration of cells from a mixture even when using a fluidic control system. Therefore, the present invention can be applied to lab-on-a-chip technology.

## Claims

1. A method of separating cells or viruses from a mixture using phase separation comprising:
suspending a sample containing cells or viruses in a kosmotropic salt solution having a pH of 3-6;
aggregating the cells or viruses by adding a cationic polymer to the suspension; adhering the cells or viruses - polymer complex to a solid substrate by phase separation of cationic polymer; and
separating the solid substrate on which the cells or viruses are adhered from the suspension,
wherein the kosmotropic salt is citrate or phosphate.

2. The method of claim 1, wherein the cationic polymer is polyamine.

3. The method of claim 2, wherein the polyamine is selected from the group consisting of polyallylamine, polyamino acid, polyethyleneimine, and polyethylimine.

4. The method of claim 1, wherein the placing the suspension in contact with the solid substrate is carried out in a static state or a fluidic state.

5. The method of claim 1, wherein the solid substrate has a planar structure, a bead structure, or a pillar structure.

6. The method of claim 1, wherein the cationic polymer separates the cells or viruses from the mixture by coupling to or mixing with the solid substrate.

7. The method of claim 1, wherein the cationic polymer is initially soluble and aggregate together with cells when added to the kosmotropic salt - based solution, thereby causing phase separation.

8. A kit for separating cells or viruses from a mixture comprising:
a cationic polymer;
a kosmotropic salt solution having a pH of 3-6; and
a solid substrate to which the cell-cationic polymer complex adheres,
wherein the kosmotropic salt is citrate or phosphate.

9. The kit of claim 8, wherein the solid substrate has a planar structure, a bead structure, or a pillar structure.

10. The kit of claim 8, wherein the cationic polymer is polyamine.

11. The kit of claim 10, wherein the polyamine is selected from the group consisting of polyallylamine, polyamino acid, polyethyleneimine, and polyethylimine.

## Patentansprüche

1. Verfahren zum Abtrennen von Zellen oder Viren von einer Mischung unter Verwendung einer Phasentrennung, umfassend:
das Suspendieren einer Probe, die Zellen oder Viren enthält, in einer Lösung eines kosmotropen Salzes mit einem pH von 3-6;
das Aggregieren der Zellen oder Viren durch Zugeben eines kationischen Polymers zu der Suspension;
das Anhaften des Zellen- oder Viren-Polymer-Komplexes an ein festes Substrat durch Phasentrennung des kationischen Polymers; und
das Abtrennen des festen Substrats, an welchem die Zellen oder Viren haften, von der Suspension,
wobei das kosmotrope Salz ein Citrat oder Phosphat ist.

2. Verfahren nach Anspruch 1, wobei das kationische Polymer ein Polyamin ist.

3. Verfahren nach Anspruch 2, wobei das Polyamin ausgewählt ist aus der Gruppe bestehend aus Polyallylamin, Polyaminosäure, Polyethylenimin und Polyethylimin.

4. Verfahren nach Anspruch 1, wobei das Inkontaktbringen der Suspension mit dem festen Substrat in einem statischen Zustand oder einem fluiden Zustand erfolgt.

5. Verfahren nach Anspruch 1, wobei das feste Substrat eine planare Struktur, eine Perlenstruktur oder eine Säulenstruktur aufweist.

6. Verfahren nach Anspruch 1, wobei das kationische Polymer die Zellen oder Viren durch Koppeln an das feste Substrat oder Vermischen mit dem festen Substrat von der Mischung abtrennt.

7. Verfahren nach Anspruch 1, wobei das kationische Polymer anfänglich löslich ist und zusammen mit den Zellen aggregiert, wenn es zu der Lösung auf Basis des kosmotropen Salzes zugegeben wird, wodurch eine Phasentrennung hervorgerufen wird.

8. Kit zum Abtrennen von Zellen oder Viren von einer Mischung, umfassend:
ein kationisches Polymer,
eine Lösung eines kosmotropen Salzes mit einem pH von 3-6; und
ein festes Substrat, an welchem der Komplex aus Zellen und kationischem Polymer haftet,
wobei das kosmotrope Salz ein Citrat oder Phosphat ist.

9. Kit nach Anspruch 8, wobei das feste Substrat eine planare Struktur, eine Perlenstruktur oder eine Säulenstruktur aufweist.

10. Kit nach Anspruch 8, wobei das kationische Polymer ein Polyamin ist.

11. Kit nach Anspruch 10, wobei das Polyamin ausgewählt ist aus der Gruppe bestehend aus Polyallylamin, Polyaminosäure, Polyethylenimin und Polyethylimin.

## Revendications

1. Procédé de séparation de cellules ou de virus à partir d'un mélange en utilisant une séparation de phases, comprenant :
la mise en suspension d'un échantillon contenant des cellules ou des virus dans une solution de sel cosmotropique ayant un pH de 3 à 6 ;
l'agrégation des cellules ou virus en ajoutant un polymère cationique à la suspension ;
l'adhésion du complexe cellules ou virus-polymère à un substrat solide par séparation des phases du polymère cationique ; et
la séparation du substrat solide sur lequel les cellules ou virus sont collés à partir de la suspension,
dans lequel le sel cosmotropique est un citrate ou un phosphate.

2. Procédé selon la revendication 1, dans lequel le polymère cationique est une polyamine.

3. Procédé selon la revendication 2, dans lequel la polyamine est choisie dans le groupe constitué par une polyallylamine, un acide polyaminé, une polyéthylèneimine et une polyéthylimine.

4. Procédé selon la revendication 1, dans lequel la mise en contact de la suspension avec le substrat solide est effectuée dans un état statique ou un état fluide.

5. Procédé selon la revendication 1, dans lequel le substrat solide a une structure plane, une structure de bille ou une structure de pilier.

6. Procédé selon la revendication 1, dans lequel le polymère cationique sépare les cellules ou les virus à partir du mélange par couplage à ou mélange avec le substrat solide.

7. Procédé selon la revendication 1, dans lequel le polymère cationique est initialement soluble et s'agrège avec les cellules lorsqu'il est ajouté à la solution à base de sel cosmotropique, provoquant ainsi une séparation des phases.

8. Kit pour séparer des cellules ou des virus à partir d'un mélange, comprenant :
un polymère cationique ;
une solution de sel cosmotropique ayant un pH de 3 à 6 ; et
un substrat solide auquel le complexe cellules-polymère cationique adhère,
dans lequel le sel cosmotropique est un citrate ou un phosphate.

9. Kit selon la revendication 8, dans lequel le substrat solide a une structure plane, une structure de bille ou une structure de pilier.

10. Kit selon la revendication 8, dans lequel le polymère cationique est une polyamine.

11. Kit selon la revendication 10, dans lequel la polyamine est choisie dans le groupe constitué par une polyallylamine, un acide polyaminé, une polyéthylèneimine et une polyéthylimine.
